# EUROPEAN PATENT SPECIFICATION

(11) **EP 2 565 157 B1**
(45) Date of publication and mention of the grant of the patent: **04.10.2017**
(21) Application number: 12190248.0
(22) Date of filing: 28.08.2002
(51) Int. Cl.: C01B 21/24, A61K 33/00, A61M 16/10, A61K 33/24, A61K 33/30, A61K 33/34, A61K 9/00, A61K 9/20, A61K 9/70, A61M 16/12

(54) **NITRIC OXIDE GENERATION**
STICKOXID-ERZEUGUNG
GENERATION D'OXYDE NITRIQUE

(30) Priority: 05.09.2001 US 316964 P
(43) Date of publication of application: 06.03.2013
(62) Divisional of application: 02773248.6
(73) Proprietor: Geno LLC, Lincoln, MA 01773 (US)
(72) Inventor: Fine, David H., Lincoln, MA 01773 (US); MacDonald, Stephen J., Salem, NH 03079 (US); Rounbehler, David, West Harwich, MA 02671 (US); Wheeler, David, Gloucester, MA 01930 (US); Rolfe, Jonathan, North Easton, MA 02356 (US); Jarvis, George, Arlington, MA 02476 (US)
(74) Representative: Howe, Steven

(56) References cited:
- WO-A1-92/10228
- US-A- 5 683 668
- US-A- 6 103 275
- DATABASE WPI Week 197809 Thomson Scientific, London, GB; AN 1978-17066A XP002720719, & NL 7 608 957 A (STAMICARBON BV) 14 February 1978 (1978-02-14)
- A. Blanchard: "Nitric Oxide [Nitrogen(II) Oxide] - Inorganic Syntheses, Volume 2 - Blanchard - Wiley Online Library", , 1 January 1946 (1946-01-01), XP055103356, Retrieved from the Internet: URL:http://onlinelibrary.wiley.com/doi/10. 1002/9780470132333.ch37/summary;jsessionid =093C3A757710D3EA6A6804D66292C287.f01t03 [retrieved on 2014-02-20]
- DATABASE WPI Week 198741 Thomson Scientific, London, GB; AN 1987-290912 XP002720720, & SU 1 298 188 A (MARCHENKO G A) 23 March 1987 (1987-03-23)

## Description

### TECHNICAL FIELD

This invention relates to a method for controllably generating nitric oxide.

### BACKGROUND

Nitric oxide plays an important role in the regulation of biochemical pathways in living organisms. The inhalation of low levels (20 to 100 ppm) of nitric oxide has been shown to have a major therapeutic value in treatment of a diverse range of disorders ranging from reversible and irreversible pulmonary hypertension to treatment of neonates exhibiting hypoxemic respiratory failure and persistent pulmonary hypertension. Conventional medical uses of nitric oxide gas can involve dilution of a nitric oxide gas stream with gases immediately before administration of the nitric oxide gas to a mammal. Precise delivery of nitric oxide at therapeutic levels of 20 to 100 ppm and inhibition of reaction of nitric oxide with oxygen to form toxic impurities such as nitrogen dioxide gas is needed for effective inhalation therapy.

United States Patent No. 6,103,275 describes a simple, biocompatible system and procedure for generating nitric oxide. A mixture of powdered sodium nitrite, ascorbic acid, and maleic acid (or another organic acid of adequate strength) immediately generates nitric oxide on treatment with water.

United States Patent No. 5,683,668 describes a method for generating NO gas by exposing zwitterionic polyamine-nitric oxide adducts to suitable conditions to effect the release of NO, such as by contact with a mildly acidic solvent or water of neutral or slightly alkaline pH.

Database WPI; Week 197809; Thomson Scientific, London, GB; AN 1978-17066A; XP002720719, & NL 7 608 957 A (STAMICARBON BV), 14 February 1978 describes the production of nitrogen monoxide by contacting an aqueous solution of ammonium nitrite with a mineral acid at 0-60 degrees C whilst maintaining the pH at <3.5 and with intensive stirring.

"Nitric Oxide [Nitrogen(II) Oxide] - Inorganic Syntheses, Volume 2 - Blanchard - Wiley Online Library" by A. Blanchard describes the synthesis of nitric oxide.

Database WPI; Week 198741; Thomson Scientific, London, GB; AN 1987-290912; XP002720720, & SU 1 298 188 A (MARCHENKO G A), 23 March 1987 describes that the use of 50% H₂SO₄ (I) as the acid and formaldehyde (II) as reducer in the precipitation of NO increases the efficiency of the process.

International Patent Application with publication no. WO 92/10228 describes an inhaler device containing nitric oxide gas and/or a nitric oxide-releasing compound.

### SUMMARY

The scope of protection is defined by the independent claim, to which reference should now be made. Optional features are set out in the dependent claims.

According to the present invention, there is provided a method of delivering nitric oxide gas comprising: non-electrolytically generating first gas from a nitric oxide precursor that includes a precursor salt including contacting the nitric oxide precursor with a buffer solution to form a mixture, wherein the first gas includes nitric oxide and is substantially devoid of nitrogen dioxide; and transporting the first gas.

Nitric oxide, also known as nitrosyl radical, is a free radical that is an important signaling molecule in pulmonary vessels. Nitric oxide can moderate pulmonary hypertension caused by elevation of the pulmonary arterial pressure. Inhaling low concentrations of nitric oxide, for example, in the range of 20-100 ppm can rapidly and safely decrease pulmonary hypertension in a mammal by vasodilation of pulmonary vessels.

Some disorders or physiological conditions can be mediated by inhalation of nitric oxide. The use of low concentrations of inhaled nitric oxide can prevent, reverse, or limit the progression of disorders which can include, but are not limited to, acute pulmonary vasoconstriction, traumatic injury, aspiration or inhalation injury, fat embolism in the lung, acidosis, inflammation of the lung, adult respiratory distress syndrome, acute pulmonary edema, acute mountain sickness, post cardiac surgery acute pulmonary hypertension, persistent pulmonary hypertension of a newborn, perinatal aspiration syndrome, haline membrane disease, acute pulmonary thromboembolism, heparin-protamine reactions, sepsis, asthma and status asthmaticus or hypoxia. Nitric oxide can also be used to treat chronic pulmonary hypertension, bronchopulmonary dysplasia, chronic pulmonary thromboembolism and idiopathic or primary pulmonary hypertension or chronic hypoxia. Advantageously, nitric oxide can be generated and delivered in the absence of harmful side products, such as nitrogen dioxide. The nitric oxide can be generated at a concentration suitable for delivery to a mammal in need of treatment.

A nitric oxide delivery system can be a controlled drug delivery system, which produces steady-state levels of nitric oxide. The system can provide for zero-order, first order or second order drug release kinetics. Controlled drug delivery devices can provide a constant level of pharmaceutical drug to a mammal which can optimize the drug input rate into the systemic circulation, improve mammal compliance, minimize side effects, and maximize drug product efficacy.

Controlled nitric oxide delivery can include controlling the diffusion/dissolution of the nitric oxide. The nitric oxide precursor composition can include a matrix and a contained phase of a nitric oxide precursor, for slow or controlled release of nitric oxide into the surrounding or external medium. Controlling the release of nitric oxide can result in greater longevity of the nitric oxide precursor and longer availability of the nitric oxide precursor for its intended purpose while providing a means for greater control in the concentration of nitric oxide into the surrounding medium.

A disclosed apparatus for delivering a therapeutic gas including nitric oxide includes a receptacle including a therapeutic gas outlet and a non-electrolytic nitric oxide precursor receiver and a transport gas inlet fluidly communicating from a source of a transport gas to the therapeutic gas outlet through the non-electrolytic nitric oxide precursor receiver. The therapuetic gas delivery system can be fluidly connectable to the therapeutic gas outlet. The therapeutic gas delivery system can include a gas purifier which can be a filter. The therapeutic gas delivery system can include a mask fluidly connectable to the therapeutic gas outlet that can be connectable to a mammal.

Also disclosed is a method of delivering nitric oxide to a mammal that includes non-electrolytically generating a therapeutic gas from a nitric oxide precursor, wherein the therapeutic gas includes nitric oxide and is substantially devoid of nitrogen dioxide and transporting the therapeutic gas to a mammal. Non-electrolytically generating the therapeutic gas can include contacting the nitric oxide precursor with a buffer solution to form a mixture. The buffer solution can be a pH buffer combination. The nitric oxide precursor can be a nitrite salt. The nitrite salt can be, for example, sodium nitrite. The transport gas can be swept over the mixture. The therapeutic gas can deliver, for example, 20 to 60 ppm nitric oxide to the mammal. The transport gas can be oxygen, ambient air, or a mixture of air and oxygen. The nitric oxide can be released from the precursor for over at least an hour.

A disclosed kit includes a nitric oxide precursor and instructional material describing a method of generating a therapeutic gas and transporting the therapeutic gas, the therapeutic gas comprising nitric oxide and being substantially devoid of nitrogen dioxide. The nitric oxide precursor can be a nitrite salt. The nitrite salt can be, for example, sodium nitrite. The nitric oxide can be released from the precursor for over at least an hour.

A disclosed nitric oxide generating composition includes a nitric oxide precursor contained within a matrix, the matrix being non-reactive with the nitric oxide precursor. The matrix can be a hydrogel, for example, a urethane. The matrix can be a hydrophilic polymer, for example, a polysaccharide. The nitric oxide precursor can be a nitrite salt which can be sodium nitrite. The matrix can be in a shape which can include a sphere, a monolith or a three-dimensional object. The three-dimensional object can be a cylinder or a film. The matrix can further include an additive, for example, a polymer, a salt, a filler or a solvent.

The hydrogel can include a polymer. The polymer can be a urethane, a polysaccharide, a polyphosphazene, a polyacrylate, a block copolymer, a polyethylene oxide-polypropylene glycol block copolymer, a fibrin, a polyvinylpyrrolidone, a hyaluronic acid, a collagen or a polyethylene glycol.

The hydrophilic polymer can include a component which can be guar gum, gum arable, gum karaya, gum ghatti, locust bean gum, tamarind gum, agar, agarose, carageenan gum, pectin or gluten.

A disclosed method of administering nitric oxide to a mammal includes generating a therapeutic gas including nitric oxide from a nitric oxide precursor contained in a matrix that is non-reactive with the precursor and transporting the therapeutic gas in a transport gas stream to the mammal. The nitric oxide precursor is contacted with, for example, a reaction solution to form a mixture.

The reaction solution can include a pH buffer combination. The pH buffer combination can be acetic acid/acetate, hydrochloric acid/chloride, hydrochloric acid/citrate, citric acid/phosphate, phosphoric acid/phosphate or citric acid/citrate. The pH of the mixture can be in the range of 4 to 7 or 6.5 to 6.9. The reaction solution can include a nitric oxide releasing salt, for example, a ferrous salt.

Also disclosed is a unitary structure that includes a mixture of a matrix and a plurality of nitric oxide precursor particles contained in the matrix.

Also disclosed is a method of manufacturing a unitary structure for delivering nitric oxide that includes combining a matrix and a plurality of nitric oxide precursor particles to form a mixture and shaping the mixture to form a unitary structure.

Also disclosed is a process for preparing a formulation for delivering nitric oxide that includes combining a plurality of the nitric oxide precursor particles within a hydrophilic matrix.

Also disclosed is an electrophoresis apparatus for delivering nitric oxide to a patient that includes a delivery portion, a reaction chamber in fluid communication with the delivery portion and a nitric oxide precursor receiving portion including an electrophoresis region bounded by a first electrode and a second electrode arranged to migrate a nitric oxide precursor to the reaction chamber when a voltage is applied across the first electrode and the second electrode.

Also disclosed is a method of producing nitric oxide that includes applying a voltage across a cavity of an electrophoresis region bound by a first electrode and a second electrode arranged to migrate a nitric oxide precursor in the electrophoresis region to a reaction chamber and contacting the nitric oxide precursor with a reaction solution in the reaction chamber to generate a therapeutic gas including nitric oxide. The method includes varying the voltage between the electrodes. The method includes varying the concentration of the nitric oxide precursor. The therapeutic gas includes nitric oxide and is substantially devoid of nitrogen dioxide.

Also disclosed is a method of delivering nitric oxide to a mammal that includes disposing a cathode and an anode in a solution of a nitric oxide precursor, applying a voltage across the cathode and anode to generate nitric oxide substantially devoid of nitrogen dioxide, contacting a transport gas with the solution of nitric oxide precursor to form a therapeutic gas; and transporting the therapeutic gas to a mammal.

Also disclosed is a method of delivering nitric oxide from an electrochemical cell to a mammal that includes disposing a cathode and an anode in a solution of dilute nitric acid, the anode including copper, applying a voltage across the cathode and the anode to generate nitric oxide substantially devoid of nitrogen dioxide; contacting a transport gas through the solution of dilute nitric acid to form a therapeutic gas including the nitric oxide and transporting the therapeutic gas to the mammal. The dilute nitric acid can be at least 0.5 M. The therapeutic gas includes nitric oxide and is substantially devoid of nitrogen dioxide.

Other features or advantages will be apparent from the following detailed description of several embodiments, and also from the appended claims.

### DESCRIPTION OF DRAWINGS

**FIG. 1** is a drawing depicting a schematic view of a nitric oxide generation and delivery system.
**FIG. 2** is a drawing depicting a schematic view of a nitric oxide generation and delivery system.
**FIG. 3** is drawing depicting a schematic view of nitric oxide precursors.
**FIG. 4** is a drawing depicting a schematic view of a nitric oxide generation and delivery system.
**FIG. 5** is a drawing depicting a schematic view of a nitric oxide generation and delivery system.
**FIG. 6** is a drawing depicting a schematic view of a nitric oxide generation and delivery system.

### DETAILED DESCRIPTION

Various nitric oxide precursors can be used in a nitric oxide delivery system. Nitric oxide precursors can include a nitrogen-containing compound with a structure X-Nitric Oxide, when X is an organic residue or a precursor salt. For example, the nitric oxide precursor can include an alkali metal nitrite, an alkaline earth metal nitrite, a transition metal nitrite or an ammonium nitrite, for example, potassium nitrite, sodium nitrite, rubidium nitrite, strontium nitrite, barium nitrite, calcium nitrite, copper nitrite, zinc nitrite, or mixtures thereof. The nitric oxide precursor can include nitrogen-containing acids, such as nitric acid. Physical characteristics of the nitric oxide precursor, such as the dissolution rate, can be used to control delivery of nitric oxide.

The nitric oxide precursor can be dissolved in a solution in which the precursor can dissociate to form anions, including nitrite anions, and cations. The solution can include a buffer solution. A buffer solution can include a pH buffer combination which is a solution containing either a weak acid or a weak base at a concentration that renders the solution resistant to change in pH. The buffer solution can provide a source of hydrogen cations, which can combine with the nitrite anions to form nitrous acid (HNO₂). Nitrous acid can decompose into several products in water. One of these products is nitric oxide. The reactions are summarized below in equations (I), (II) and (III):

NaNO₂ ↔ Na⁺ +NO₂⁻ (I)

NO₂⁻ + H⁺ ↔ HNO₂ (II)

3HNO₂ ↔ H₂O + H⁺ + NO₃⁻ + 2NO (III)

The nitric oxide precursor can include sodium nitrite, which dissociates into sodium cations and nitrite anions, as shown in equation (I). The nitrite anions in the buffer solution can form nitrous acid as shown in equation (II), which can decompose into water, nitrate and hydrogen ions and two molecules of gaseous nitric oxide, as shown in equation (III).

The generated nitric oxide gas formed by the above reactions has a low solubility in the pH buffer combination (e.g., 0.00983 g nitric oxide per liter at 0°C; 4.6 mL/100mL at 20°C in water (Merck Index, 10th Edition, 1983)). The relatively insoluble nitric oxide can be removed from the solution by a transport gas stream to form a therapeutic gas. The transport gas can be 100% oxygen, a mixture of air and oxygen, or ambient air. The transport gas stream can be bubbled, otherwise distributed through the solution or swept over the solution. Other byproducts such as, for example, nitrous acid and nitrogen dioxide, can be volatile and can be carried with the transport gas stream along with nitric oxide formed in the reaction.

When delivering nitric oxide for therapeutic use to a mammal, it can be important to avoid delivery of nitrogen dioxide to the mammal. Nitrogen dioxide can be formed by the oxidation of nitric oxide with oxygen. The rate of formation of nitrogen dioxide is proportional to the square power of the nitric oxide concentration and the single power of the oxygen concentration. Reducing the nitric oxide concentration by a factor of ten reduces the nitrogen dioxide concentration by a factor of one hundred. Thus, by reducing the nitric oxide concentration in a therapeutic gas, the therapeutic gas can be substantially devoid of nitrogen dioxide. For example, when nitric oxide concentration in the transport gas is below 100 ppm, the resulting therapeutic gas generated from the nitric oxide precursor in a solution is substantially devoid of nitrogen dioxide.

In certain circumstances, the concentration of nitric oxide generated in the therapeutic gas is controlled, for example, by the concentration of nitric oxide precursor provided to the solution, the concentration of hydrogen cations in the solution, and the characteristics of the pH buffer combination. Other factors that can affect the nitric oxide concentration in the therapeutic gas can include, for example, physical form of the nitric oxide precursor, presence of a reduction-oxidation reaction in an optional gas purifier, and rate of flow of the transport gas through the solution.

The concentrations of hydrogen cations and the nitric oxide precursor can control the rate of generation of nitric oxide. Since the concentration of nitric oxide is low, about 20 to 100 ppm, reaction conditions that increase the concentration of nitric oxide precursor and decrease the concentration of hydrogen ions lead to a stoichiometrically inefficient reaction. Decreasing the concentration of hydrogen ions, for example, by using a weak acid, shifts the equilibrium in equation (II) toward the nitrite anions. A reservoir of nitrite ions can be created such that the nitrous acid concentration is maintained at a relatively constant level.

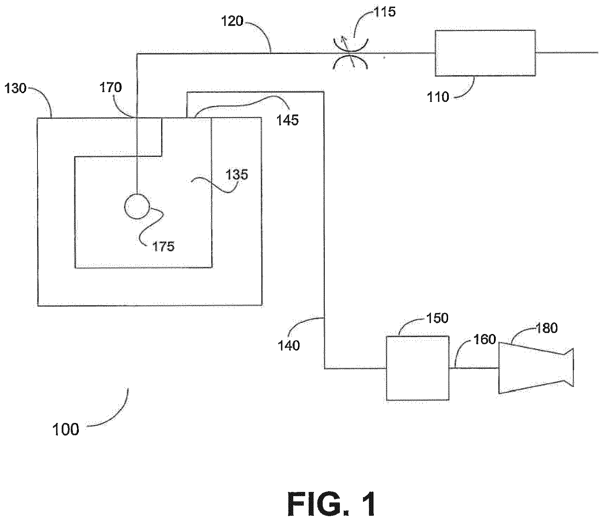

Referring to **FIG. 1****,** a nitric oxide delivery system **100** for producing a therapeutic gas including nitric oxide includes a gas flow controller **110,** a restrictor valve **115,** a tube **120,** and a receptacle **130.** A gas flow controller is a pump set to vary the flow rate of the transport gas. The gas flow controller can be a diaphragm pump. The receptacle **130** includes a non-electrolytic nitric oxide precursor receiver **135.** The non-electrolytic nitric oxide precursor receiver is a receiver that does not require application of voltage for the nitric-oxide generating reaction to proceed. The non-electrolytic nitric oxide precursor receiver includes a transport gas inlet **170** and a therapeutic gas outlet **145.** The therapeutic gas outlet **145** is connectable to a gas delivery system, which includes a tube **140,** an optional gas purifier **150,** a tube **160,** and a mask **180.** The mask **180** is connectable to a mammal. The transport gas inlet **170** includes a gas distributor **175.** The gas distributor **175** distributes the transport gas in the receiver **135.** The gas distributor **175** can be a mechanical agitator, which can include, for example, a stirrer, a vibrator, a sparger and a bubbler to prevent supersaturation of nitric oxide in the receiver **135.** The gas flow controller **110** controls flow rate of a transport gas through the receiver. For example, the flow rate can be from 1 to 10 liters per minute, 2-8 liters per minute or 2 to 5 liters per minute. The flow rate of the transport gas can be in the range of 1 to 20 liters per minute. The transport gas can be 100% oxygen, a mixture of air and oxygen, or air. The rate of flow of transport gas in the reaction vessel can affect the generation of nitric oxide. Mechanical agitation using, for example, stirring, vibration, or bubbling the transport gas through the solution, sweeping the transport gas over the solution or other methods of agitation enhances the transport of nitric oxide in the therapeutic gas.

In a general process for delivering nitric oxide, the gas flow controller **110** conveys a stream of transport gas at a specific flow rate, into and through the tube **120** and into and through the non-electrolytic nitric oxide precursor receiver **145,** which contains the nitric oxide precursor and buffer solution. The non-electrolytic nitric oxide precursor receiver **135** can be, for example, filled to half the capacity with the nitric oxide precursor and the buffer solution, for example, a pH buffer combination. The pH buffer combination can be used to control the pH of the solution very close to pH 7 to maintain a concentration of hydrogen ions suitable to control nitric oxide production from the solution. Suitable pH buffers include, for example, combinations of acetic acid and acetate salt (acetic acid/acetate), combinations of hydrochloric acid and chloride salt, combinations of hydrochloric acid and citrate salt (hydrochloric acid/citrate), combinations of citric acid and phosphate salt, combinations of phosphoric acid and phosphate salt (phosphoric acid/phosphate) and combinations of citric acid and citrate salt (citric acid/citrate). A pH within the range of 4.5-6.9, or the range of 6.5-6.9, can be maintained in the solution using the pH buffer combination.

Nitric oxide is generated in the nitric oxide precursor receiver **135.** The stream of transport gas carries the generated nitric oxide as the therapeutic gas into and through tube **140** into (optionally) a gas purifier **150.** If necessary, the therapeutic gas can pass into and through the optional gas purifier **150** which can remove any residual impurities such as nitrogen dioxide and nitrous acid, if present. The therapeutic gas including the nitric oxide is transported in the transport gas into and through tube **160** to mask **180** to the mammal. The mask **180** can include any device or implement that is used to provide the nitric oxide stream to the mammal and is typically selected by the physician based on the mammal need and condition. For example, the mask **180** can be in the form of a tight-fitting or a loose fitting mask, an intubation tube, a nasal delivery tube, or a tube that generally directs the nitric oxide gas in the region around the mammal's mouth and/or nose.

In certain circumstances, the therapeutic gas can be passed through a nitric oxide releasing solution. A nitrite releasing salt assists in the generation of nitric oxide from the nitric oxide precursor. For example, a second salt, such as a nitric oxide-releasing reactant, can be added to the solution. A nitric oxide-releasing reactant, for example, an iodide salt or ferrous salt, assists the production of nitric oxide as shown below:

2NO₂⁻ + 2I⁻ + 4H⁺ → I₂ + 2H₂O + ₂NO

or

2NO₂⁻ + 2Fe⁺² + 6ₑ⁻→ 2Fe⁺³ + 2H₂O + 2NO

For example, the nitric oxide-releasing reactant can be 1 molar ferrous sulfate solution or 10 % wt/wt aqueous solution of sodium iodide. The nitrite releasing salt can include salts of Groups I, II, III, IV, V, VI and VII of the periodic table. For example, the nitrite releasing salt can include a ferrous salt.

In certain circumstances, the therapeutic gas can be passed through an optional therapeutic gas purifier **150.** When the therapeutic gas stream contacts the optional therapeutic gas purifier, residual impurities, such as nitrous acid and nitrogen dioxide, are removed from the therapeutic gas stream. The optional gas purifier can include a filter, for example, a semi-permeable membrane or barrier, a scrubbing solution, a reduction-oxidation solution, or a pyrolizer. The semi-permeable membrane is a barrier which allows the nitric oxide to pass and retains the impurities. The scrubbing solution is a solution that removes impurities by neutralizing them, for example, a solution of 10% sodium bicarbonate, a 1M ferrous salt solution or an acidified 1M ferrous sulfate solution. A series of aqueous reservoirs can be used to completely decompose the nitrous acid and dissolve any nitric acid or nitrogen dioxide impurities. The reduction-oxidation solution contains a reduction-oxidation agent, which converts impurities completely into nitric oxide. The reduction-oxidation agent can include a ferrous salt. The pyrolizer is a chamber or other component which decomposes the impurities such as nitrous acid and nitrogen dioxide by irradiation or heating. A catalyst, for example, platinum, nickel or silver, can be used to decrease the pyrolysis temperature. For example, the impurities such as nitrous acid and nitrogen dioxide can be passed through a 12 inch long silver tube, 1/8 inch in diameter, heated at 800°C at a flow rate of 1L/minute. The removal of impurities can be enhanced by using a convoluted or a long path for the bubbling of the therapeutic gas stream through the filter. Additionally, the surface-to-volume ratio of the bubbles can be increased for effective filtration of impurities. For example, a gas sparger can be used to make smaller bubbles. Alternatively, filter media can also be coated onto a filter or walls of a tube, which can produce a dry therapeutic gas stream upon filtration

A detector can be included in the therapeutic gas delivery system to detect the concentration of nitric oxide in the therapeutic gas stream. The detector can also detect the concentration of nitrogen dioxide in the therapeutic gas, if necessary, and may provide a warning if the nitric oxide concentration is outside a predetermined range or if the concentration of nitrogen dioxide is above a threshold value. Examples of monitoring techniques include chemiluminescence and electrochemical techniques, and are discussed in, for example, in Francoe et al., "Inhaled nitric oxide: Technical Aspects of Administration and Monitoring," Critical Care Medicine, 24(4): 782-796 (1998), which is incorporated by reference in its entirety. The presence of nitric oxide can be detected by for example, a modified version of a Thermo-Electron chemiluminescence (CL) detector.

A therapeutic gas can contain at least 1 ppm of nitric oxide. The therapeutic gas can include less than 100 ppm of nitric oxide. For example, the nitric oxide concentration in the therapeutic gas can be from 20 to 100 ppm. The nitric oxide can be released from the precursor over a period of time ranging from 1 minute to 7 days, 2 days to 3 days, or two hours to twenty four hours.

Oxidation-reduction reactions can assist in the production of nitric oxide. For example, a second salt, such as a nitric oxide-releasing reactant, can be added to the solution. A nitric oxide-releasing reactant, for example, an iodide salt or ferrous salt, assists the production of nitric oxide as shown below:

2NO₂⁻ + 2I⁻ + 4H⁺ → I₂ + 2H₂O + 2NO

or

2NO₂⁻ + 2Fe⁺² + 6ₑ⁻→ 2Fe⁺³ + 2H₂O + 2NO

For example, the nitric oxide-releasing reactant can be a 1 molar ferrous sulfate solution or a 10 % wt/wt aqueous solution of sodium iodide.

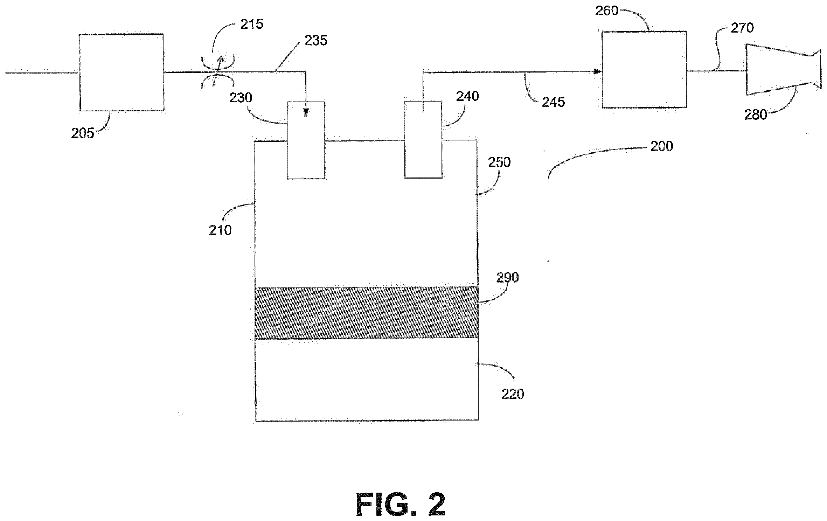

Referring to **FIG. 2****,** a nitric oxide delivery system **200** for producing a stream of nitric oxide includes a transport gas pump **205,** a restrictor valve **215,** a gas inlet tube **235,** a gas inlet **230** and nitric oxide precursor receiver **210.** The nitric oxide precursor receiver **210** includes nitric oxide precursor contained in a matrix **220** and a reaction solution **290.** The reaction solution can include the pH buffer combination which can be used to control the pH of the solution to very close to pH 7 to maintain a concentration of hydrogen ions suitable to control nitric oxide production from the solution. Suitable pH buffers include, for example, combinations of acetic acid and acetate salt (acetic acid/acetate), combinations of hydrochloric acid and chloride salt, combinations of hydrochloric acid and citrate salt (hydrochloric acid/citrate), combinations of citric acid and phosphate salt, combinations of phosphoric acid and phosphate salt (phosphoric acid/phosphate) and combinations of citric acid and citrate salt (citric acid/citrate). A pH within the range of 4.5-7.0, or the range of 6.5-6.9, can be maintained in the solution using the pH buffer combination. The nitric oxide precursor receiver includes a gas outlet **240** connectable to a tube **245,** an optional gas purifier **260,** a tube **270** and a mask **280.** The mask **280** is connectable to a mammal. The restrictor valve **215** and the transport gas pump **205** control the flow rate of the transport gas. The flow rate of the transport gas can be controlled by, for example, a restrictor valve **215.**

Referring to **FIG. 2****,** in a general process for delivering nitric oxide, the gas flow controller **205** conveys a stream of transport gas at a specific flow rate into and through the inlet **230** and into and through the non-electrolytic nitric oxide precursor receiver **210** which contains the nitric oxide precursor contained in a matrix **220** and reaction solution **290.** Nitric oxide is generated in the nitric oxide precursor receiver **210.** The stream of transport gas transfers the generated nitric oxide in the therapeutic gas through the gas outlet **240** and through tube **245** into optional gas purifier **260,** if necessary. The therapeutic gas including the nitric oxide, is transported in the transport gas into and through tube **270** to mask **280** to the mammal.

The matrix can be a non-reactive support in which the nitric oxide precursor can be contained. The nitric oxide precursor diffuses from the matrix when the matrix, for example, swells, dissolves or erodes in the reaction solution. With changes in these morphological characteristics of the matrix, the mobility of segments in the matrix changes which affects diffusivity of the nitric oxide precursor. Addition of other additives, for example, a polymer, a filler, a solvent, or modifying reaction factors such as ionic strength, temperature or pH can alter the intermolecular forces, free volume, or glass transition temperature of the matrix, and, consequently, can alter the diffusivity of the nitric oxide precursor. A filler is a substance added to a product to add bulk, weight, viscosity, or strength. The matrix can include nitrite-releasing salt. A nitrite releasing salt assists in the generation of nitric oxide from the nitric oxide precursor. The nitrite releasing salt can include salts of Groups I, II, III, IV, V, VI and VII of the periodic table. For example, the nitrite releasing salt can include a ferrous salt. A nitric oxide precursor can be contained within the matrix, using a number of techniques. Examples of methods for containing include solvent evaporation, spray drying, solvent extraction and other methods.

The matrix can be, for example, a hydrogel. The hydrogel is a substance which is formed when an organic polymer (natural or synthetic) is cross-linked via covalent, ionic, or hydrogen bonds to create a three-dimensional open-lattice structure, which entraps water molecules to form a gel. The hydrogel can be formed from a polymer, which can include an ionically crosslinkable polysaccharide, synthetic biodegradable, biocompatible polymer, or a protein. Examples of materials which can be used to form the hydrogel can include, for example, a urethane, a polysaccharide such as alginate, a polyphosphazene, a polyacrylate, which are crosslinked ionically, a block copolymer such as PLURONICS^{™} or TETRONICS^{™}, a polyethylene oxide-polypropylene glycol block copolymer, a polyethylene glycol, or polyethylene glycol which can be crosslinked by temperature or pH. The urethane can be for example, TECOPHILIC, which is a high moisture absorption aliphatic polyether-based polyurethane (commercially available from Thermedics Corporation, Woburn, MA). The urethane can include a flexible segment which can be a highly hydrophilic compound, for example, polyethylene glycol or polypropylene glycol. The water soluble flexible segment can be immobilized when bound into the polyurethane molecule to form a polyurethane hydrogel. The polyurethane hydrogel does not fully dissolve, but swells upon hydration to form a gel. The polyurethane hydrogel can absorb two hundred times its weight in water without collapsing. The hydrogel can have a molecular weight of more than 400 g/mol, less than 1 million g/mol or between 400 and 500,000 g/mol, or between 500 g/mol and 3000 g/mol. Other materials can include proteins such as a fibrin, polymers such as a polyvinylpyrrolidone, a urethane, a hyaluronic acid, or a collagen. For example, urethane can be added to provide rigidity to the hydrogel to allow a stronger fabricated shape. For example, a urethane, TECOFLEX 80A solution grade aliphatic urethane (commercially available from Thermedics, Woburn, MA) can be added to the hydrogel to retain the post-hydration state of hydrogel and allow longer release times for the nitric oxide. In general, these polymers are at least partially soluble in aqueous solutions, such as water, buffered solutions, or aqueous alcohol solutions. The polymers can have charged side groups. These polymers are either commercially available or can be synthesized using known methods. See, for example, *"*Concise Encyclopedia of Polymer Science and Polymeric Amines and Ammonium Salts," E. Goethals, editor (Pergamen Press, Elmsford, N.Y. 1980).

The matrix can be, for example, a hydrophilic polymer. A hydrophilic polymer can be a polysaccharide containing several sugars with alternating monomer structures and can optimally contain uronic acids. Suitable hydrophilic polymers include guar gum, gum arabic, gum karaya, gum ghatti, locust bean gum, tamarind gum, agar, agarose, carageenan gum, pectin or gluten. The nitric oxide precursor can be contained into the hydrophilic polymer at different loading doses. The loading dose can be more than 0.1% or less than 25%. For example, the loading dose can be 15% of nitric oxide precursor in the hydrophilic polymer. The matrix and particles of nitric oxide precursor can be directly shaped into, for example, a tablet. The tablet can range in size from 0.01 cm diameter to 5 cm diameter. The tablet can in the range of 0.10 cm to 5 cm in thickness. The tablet can weigh 1 mg to 500 mg. The tablet can range in size from 1 cm diameter X 0.5 cm thick and weigh 300 mg. This delivery system can release nitric oxide in a controlled manner over a long time period while also achieving complete dissolution.

Referring to **FIG. 2****,** the matrix can be an agar gel matrix containing 5% (wt/wt) of sodium nitrite that can be placed in the nitric oxide precursor receiver **210.** A reaction solution **290** can be placed on the agar gel matrix. The transport gas can flow through and into the nitric oxide precursor receiver and carry the generated nitric oxide gas into gas outlet **240** as the therapeutic gas. The therapeutic gas can pass into and through the optional gas purifier **260** to remove any impurities, if present, through and into tube **270** through mask **280** to the mammal.

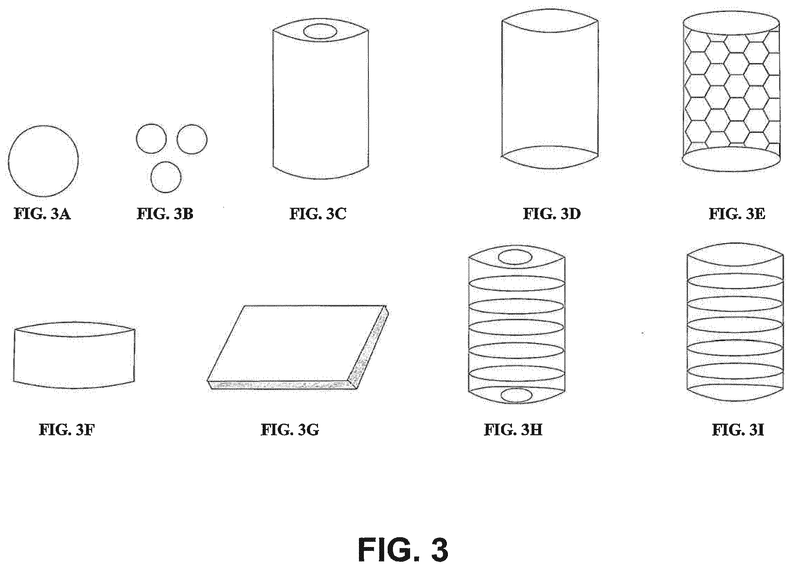

Referring to **FIG. 3****,** a matrix can include, for example, shapes that physically immobilize the nitric oxide precursor to control reaction rate of the generation of the nitric oxide. The shape of the matrix can vary depending, for example, on the type of reaction system employed. Examples of the shapes of the matrix include a sphere, a monolith or a three dimensional object. The sphere can include a ball **(****FIG. 3A****)**, a pebble **(****FIG. 3B****),** a microsphere or a pellet. A monolith is a matrix shaped into a column or column containing cells. The monolith can include a single monolith **(****FIG. 3D****),** a stacked monolith **(****FIG. 3H** and **FIG. 3I****)** or honeycombed monolith **(****FIG. 3E****).** A three dimensional object can include a tube **(****FIG. 3H****)**, a cylinder **(****FIG. 3D****),** a cake **(****FIG. 3F****),** a powder, a film **(****FIG. 3G****),** an extrudate or a granule. The body geometry and shape of the matrix will be dictated by the circumstances of use. A unitary structure for delivering nitric oxide gas can include a mixture of a matrix and a plurality of nitric oxide precursor particles contained in the matrix. The matrix can include polymers, buffers, salts, fillers or solvent as described above.

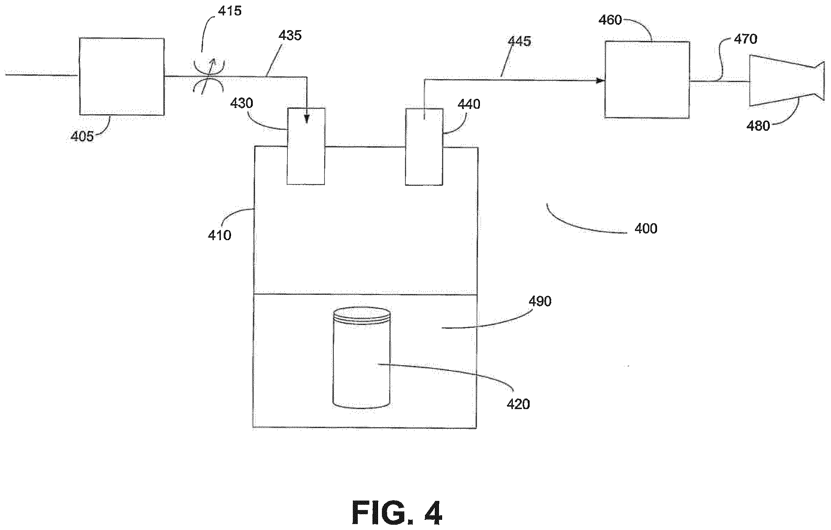

Referring to **FIG. 4****,** a nitric oxide delivery system **400** for producing a stream of nitric oxide includes a gas flow controller **405,** a restrictor valve **415,** a nitric oxide precursor receiver **410** with the monolith **420** and a reaction solution **490.** The nitric oxide precursor receiver includes a gas inlet tube **435,** gas inlet **430,** gas outlet **440** connectable to a gas outlet tube **445,** an optional gas purifier **460,** a tube **470** and a mask **480.** The mask **480** is connectable to a mammal. The gas flow controller **405** and the flow restrictor valve **415** control the flow rate of a transport gas.

Referring to **FIG. 4****,** in a general process for delivering nitric oxide, the gas flow controller **405** conveys a stream of transport gas at a specific flow rate, into and through the inlet tube **430** and into and through the nitric oxide precursor receiver **410,** which contains the monolith **420** and reaction solution **490.** The monolith **420** can include, for example, the nitric oxide precursor or a mixture of the contained nitric oxide precursor and the nitric oxide releasing reagent contained in a matrix. Water, as reaction solution **490,** can be introduced into the nitric oxide precursor receiver **410,** which initiates the generation of therapeutic gas. The transport gas flows over the monolith **420** and transfers the nitric oxide generated in the center of the monolith. A plurality of monoliths can be stacked. For example, a two-component monolith can be constructed with the nitric oxide precursor (e.g. sodium nitrite) in a first monolith and a second monolith containing the nitric oxide releasing reactant (e.g. ferrous sulfate). The two-component monolith can be placed in the reaction solution to generate low concentrations of nitric oxide. The stream of transport gas transfers the generated nitric oxide as the therapeutic gas into and through tube **440** into an optional gas purifier **460,** if necessary. The therapeutic gas including the nitric oxide is then transported in the transport gas into and through tube **470** to mask **480** to the mammal.

In another approach, controlled-release and delivery of nitric oxide can involve generation of nitric oxide by, for example, electrophoresis. An electrophoresis slab can be filled with an electrophoresis medium, and the fluid medium can be covalently cross-linked or temperature-solidified to form a gel separation medium. A sample can be loaded into a well in the slab gel, and an electric field can be generated to draw the samples through the medium. Electrophoretic migration can depend predominantly on molecular size or on a combination of size and charge.

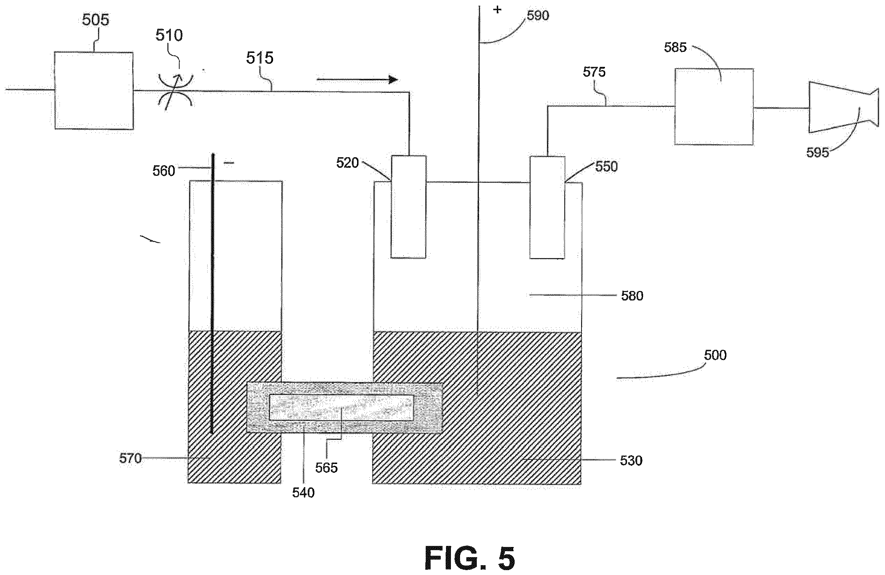

Controlled-release and delivery of nitric oxide can include, for example, an electrophoresis cell. This does not fall within the scope of the claims. Referring to **FIG. 5****,** a nitric oxide generation system **500** can include a nitric oxide receiving portion **540,** a delivery portion **580,** a first electrode **560,** and a second electrode **590.** The delivery portion **580** can include a reaction chamber **530** including a reaction solution. The nitric oxide receiving portion **540** includes a cavity **565.** The generation system **500** can include gas flow controller **505,** and an optional gas purifier **585.** The gas flow controller **505** includes a flow restrictor **510** which controls flow rate of a transport gas. The generation system **500** includes an inlet gas tube **515,** an inlet **520,** outlet **550,** and an outlet tube **575.** The outlet tube **575** can be connected to an optional gas purifier, which is connectable to a mask **595.**

In a general process for generating a stream of nitric oxide, a nitric oxide precursor salt is placed in the nitric oxide receiving portion **540.** A reaction solution can be placed in the reaction chamber **530.** A voltage can be applied across the first and second electrode, under conditions effective to cause a plurality of nitric oxide precursors to migrate to the reaction chamber **530.** The migrated nitric oxide precursors contact the reaction solution in reaction chamber **530** to generate nitric oxide. The transport gas flows from the gas flow controller **505** through the restrictor valve **510,** into and through the inlet **520** and transfers the generated nitric oxide in the headspace of **580** as therapeutic gas through and into outlet **550,** into outlet tube **575** to an optional gas purifier **585.** The optional gas purifier **585** can remove any impurities, if any. The therapeutic gas can be transported from the optional gas purifier **585** through mask **595** to the mammal.

A variety of polymeric materials can be used in the electrophoresis region to separate nitric oxide precursors. The polymeric materials can include linear polyacrylamides, polyethylene oxides, dextrans, polyethylene glycols, agar, agarose or polyvinyl alcohols. The appropriate concentration and size of the polymer material included in the medium can depend at least in part on the physical properties and complexity of the sample being analyzed, the properties of the selected polymer(s), and the desired delivery rate.

Controlled-release delivery systems can include chemical reactions, for example, generation of nitric oxide by an electrochemical cell. For example, the reaction of potassium nitrite and potassium nitrate with chromium oxide forms potassium chromate and nitric oxide. This reaction can be controlled to generate nitric oxide.

3KNO₂ + KNO₃ + Cr₂O₃ → 2K₂CrO₄ + 4NO.

Alternatively, nitric oxide can be generated, for example, by the chemical reduction reactions. Metallic copper, for example, and dilute nitric acid can react to generate nitric oxide. For every six electrons transferred, three copper cations are reduced and two molecules of NO gas are generated. The reaction is shown below:

3Cu + 8HNO₃ → 2NO + 3Cu(NO₃)₂ + 4H₂O

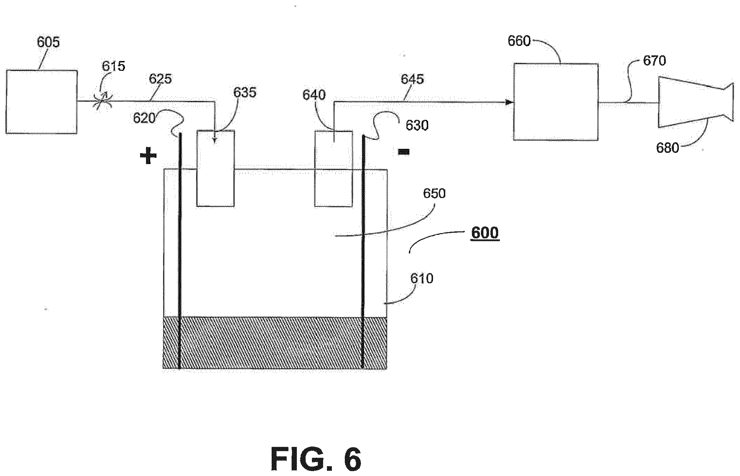

Referring to **FIG. 6****, a** nitric oxide generation and delivery system **600** (that does not fall within the scope of the claims) includes a gas controller **605** with a restrictor valve **615,** an electrochemical cell **610** with a cathode **620,** an anode **630,** an inlet tube **625,** an inlet **635,** an outlet tube **645,** an outlet **640** and a headspace **650.** The cell **610** is connected to an optional gas purifier **660,** a tube **670** and a mask **680.** The cell **610** is filled with a weak aqueous solution of nitric oxide precursor salt. The position of the anode and cathode in the cell can be modified.

In a general process for generating a stream of nitric oxide, the cathode **620** and anode **630** are immersed in the aqueous solution of nitric oxide precursor and the headspace **650** can be flushed with the transport gas. Various nitric oxide precursors can be used in the nitric oxide delivery system as described above. The concentration of the precursor salt can be 0.1% to 50%, 1% to 35% or 1% to 20% by weight. A voltage is applied between the cathode and anode to generate the nitric oxide. The transport gas is swept over the solution of nitric oxide precursor to generate nitric oxide. The nitric oxide generated can be transported with the transport gas stream as a therapeutic gas to an optional gas purifier **660.** The optional gas purifier **660** removes any impurities such as nitrogen dioxide and nitrous acid as well as any other residual impurities, if present, as described above. The purified therapeutic gas can be transported through tube **670** to mask **680** to the mammal. Several parameters affect the production of nitric oxide in this process. The reaction conditions, for example, electrode material, applied voltage, applied current and nitric oxide precursor concentration can be controlled to generate 10 to 100 ppm of NO in the therapeutic gas.

Another design variation which achieves controlled-release delivery includes delivery of nitric oxide by reaction of copper in a dilute nitric acid solution, the nitric oxide precursor, in which case anode **630** can include copper and cell **610** can be filled with, for example, an aqueous solution of dilute nitric acid as the nitric oxide precursor. The dilute nitric acid can be 0.5 M or more in concentration of nitric acid in water. Unexpectedly, the nitric oxide generated as a therapeutic gas is free of impurities such as nitrogen dioxide and nitrous acid.

The following examples describe nitric oxide generation.

### EXAMPLE 1

Using an apparatus depicted in **FIG. 1****,** a pH buffer combination (100mL) was prepared which was 1M acetic acid and 1M acetate with a pH of 4.9 and added to a non-electrolytic nitric oxide precursor receiver. Twenty grams of sodium nitrite (approximately 2M) was added to the receiver and the mixture was stirred at room temperature. A gas flow controller equipped with a restrictor valve was used to establish a flow rate of 2 liters per minute of ambient air at 20°C. The transport gas swept the headspace of the nitric oxide receiver to generate the therapeutic gas. The output of nitric oxide generated was 50 ppm in the therapeutic gas, which remained constant at 50 ppm + / - 20 for five hours.

### EXAMPLE 2

Using an apparatus depicted in **FIG. 1****,** a pH buffer combination (100 mL) was prepared which was 3M acetic acid and 3M acetate with a pH of 4.9 was added to a non-electrolytic nitric oxide receiver. Twenty grams of sodium nitrite (approximately 2M) was added to the receiver and the reaction was stirred at room temperature. A gas flow controller equipped with a restrictor valve was used to establish a flow rate of 2 liters per minute of ambient air at 20°C. The transport gas swept the headspace of the nitric oxide receiver to generate the therapeutic gas. The output of nitric oxide generated was 50 ppm in the therapeutic gas, which remained constant at 50 ppm + / - 20 for nine hours.

### EXAMPLE 3

Using an apparatus depicted in **FIG. 1****,** a solution was prepared in a non-electrolytic nitric oxide precursor receiver, by dissolving 28 grams of sodium hydrogen phosphate (Na₂HPO₄) in 100 mL of water. Sodium nitrite (15 g) was added to the solution, followed by addition of 40 g of sodium hydrogen phosphate until the solution became clear. The total volume of the solution was adjusted to 150 mL. The pH of the solution was approximately 4. In the optional gas purifier, a 0.1 mole solution of ferrous sulfate (FeSO₄) was prepared in 100 mL of water. A gas flow controller equipped with a restrictor valve was used to establish a flow rate of 2 liters per minute of ambient air at 20°C. The transport gas swept the headspace of the nitric oxide receiver to generate the therapeutic gas. The output of nitric oxide generated in the therapeutic gas was 60 ppm in the therapeutic gas, for a period of seven days.

### EXAMPLE 4

Using an apparatus depicted in **FIG. 1****,** a pH buffer combination and nitric oxide precursor mixture was prepared by adding 0.1 mole sodium phosphate monobasic, 0.1 mole sodium phosphate dibasic and 20g of sodium nitrite to 100 mL of water. The solution was 5M sodium phosphate monobasic and 2M sodium phosphate dibasic and contained 20 grams of sodium nitrite. The pH of the solution was approximately 5.6. A gas flow controller equipped with a restrictor valve was used to establish a flow rate of 2 liters per minute of ambient air at 20°C.. The transport gas was swept over the headspace of the nitric oxide precursor receiver to produce 1% nitric oxide and 99% nitrous acid gas stream. The gas stream was bubbled through an optional gas purifier, which contained a 1M sulfuric acid and 1M ferrous sulfate solution by mixing 0.1 mole of sulfuric acid and 0.1 mole of ferrous sulfate to 100 mL of water. The output of nitric oxide generated was constant in the therapeutic gas, for a period of several days.

### EXAMPLE 5

Using an apparatus depicted in **FIG. 1****,** an aqueous solution of 2 grams of sodium nitrite, and pH buffer combination of 0.1 mole acetic acid and 0.1 mole sodium acetate was prepared in 100mL of water. The solution was added to a non-electrolytic nitric oxide precursor receiver and the mixture was stirred. A diaphragm pump equipped with a restrictor valve established a flow rate of 2 liters per minute of ambient air at 20°C. The transport gas swept the headspace of the nitric oxide receiver to generate the therapeutic gas. The output of nitric oxide generated was 50 ppm in the therapeutic gas, which remained constant at 50 ppm + / - 20 for five hours.

### EXAMPLE 6

Using the apparatus depicted in **FIG. 1****,** a pump equipped with a restrictor valve was used to supply 20 °C ambient air at a flow rate of 2 L/min. Sodium nitrite (15 g) was added to the receiver containing 100 mL of an aqueous solution of a buffer including 28 g of sodium hydrogen phosphate and 40 g of sodium phosphate monobasic. The reaction was stirred at room temperature (18-22°C). The transport gas, air was swept over the headspace of the receiver into a gas purifier containing nitric oxide releasing agent 100 mL of 1molar iron(II)sulfate (FeSO₄) and 4 mL of H₂SO₄ to remove impurities.

### EXAMPLE 7

Using the apparatus depicted in **FIG. 1****,** a pump was equipped with a restrictor valve to supply 20 °C ambient air at a flow rate of 2 L/min. An aqueous solution (100 mL) was prepared of 15 g of the nitric oxide precursor sodium nitrite and a buffer consisting of 28 g of sodium phosphate (Na₂HPO₄) and 40 g of sodium phosphate monobasic (NaH₂PO₄) was placed in the receiver. The reaction was stirred at room temperature (18-22°C). The transport gas, air, was swept over the headspace of the receiver into a gas purifier containing nitric oxide releasing agent as shown in Table 1.

**Table 1**

| **Experiment** | **Nitric oxide releasing agent** |
|---|---|
| Experiment 1 | 100 mL water, 5 g NaI, 2 mL H₂SO₄ |
| Experiment 2 | 100 mL water + 5 g NaI + 2 mL H₂PO₄. |
| Experiment 3 | 100 mL Potassium Biphthalate/hydrochloric acid pH 3 buffer + 5 g NaI |
| Experiment 4 | 100 mL 1 M NaH₂PO₄ + 5 g NaI. |

### EXAMPLE 8

Referring to **FIG. 2****,** a nitric oxide precursor, sodium nitrite and nitric oxide releasing salt, ferrous sulfate were dried at 110°C for 18 hours, combined with 10% fumed silica (Cab-O-Sil M5) and ground to a particle size of 5-10 microns. A hydrogel, urethane resin was dissolved in tetrahydrofuran to produce a lacquer of 30% solids. The finely powdered nitric oxide precursor and nitric oxide releasing salt mixture were suspended in the hydrogel, urethane, of molecular weight 3000 g/mol or 500 g/mol at 20% concentration by weight of the mixture to the urethane to form a solution. The solution is cast in shallow dishes and allowed to dry at 30°C for several hours. The film was removed from the dish and stored in a dessicator. Sample films were prepared from dried ferrous sulfate, ferrous sulfate as the septahydrate, sodium nitrite and a mixture of sodium nitrite and ferrous sulfate. The reactant concentration was 10 milligrams per square centimeter of film. Varying the length of the hydrogel strips controlled the nitric oxide production. The hydrogel with reactants can be added to a solution containing reactants to release the nitric oxide as shown in Table 2.

**TABLE 2**

| **Hydrogel with reactants** | **Reaction Solution** |
|---|---|
| Hydrogel with nitric oxide precursor | Nitric oxide releasing salt in water |
| Hydrogel with nitric oxide releasing salt | Nitrite reactant in water |
| Hydrogel with both nitric oxide precursor and nitric oxide releasing salt | Water |
| Hydrogel I with nitric oxide precursor and Hydrogel II with nitric oxide releasing salt | Water |

### EXAMPLE 9

Referring to **FIG. 2****,** an agar gel solution was prepared by dissolving 1 g of Agar powder in 100 mL of boiling water, followed by addition of 5 g of nitric oxide precursor, sodium nitrite. The agar solution was cooled overnight. The agar solution (20 mL) was poured into the nitric oxide precursor and subjected to dissolution study at various reaction conditions as shown in Table 3. Controlled generation of nitric oxide was observed at all reaction conditions. The generation was slower at a pH of 3 as compared to pH of 1. The addition of FeSO₄, showed an acceleration in the production of nitric oxide in pH 1 HCl/KCl buffer solution. The nitric oxide rate increased as a function of increasing FeSO₄ concentration from 1%, 3% to 5% of FeSO₄. Nitric oxide levels were between 10 and 100 ppm in the therapeutic gas.

**TABLE 3**

| **Experiment** | **Reaction solution** |
|---|---|
| Experiment 1 | pH 3 phosphate buffer |
| Experiment 2 | pH 1, HCl/KCl buffer |
| Experiment 3 | pH 1 HCl/KCl buffer, 10% FeSO₄ |
| Experiment 4 | 1% FeSO₄ |
| Experiment 5 | 3% FeSO₄ |
| Experiment 6 | 10% FeSO₄ |

### EXAMPLE 10

Referring to **FIG. 5****,** an agarose gel is doped with a nitric oxide precursor, placed between two buffer solutions and subjected to an applied electrical field as follows. A buffer solution of 50X Tris acetic acid EDTA (TAE) is prepared by adding 242 g Tris base and 57.1 g glacial acetic acid to 100 mL of 0.5 molar EDTA to form a solution. The buffer solution (2 mL) is diluted with 98 mL water to make a solution, 1X TAE. A 1% agarose solution is prepared by adding 5 g sodium nitrite and 0.7 g agarose to 100 mL of 1X TAE at 100°C, while stirring to allow complete dissolution. The solution is allowed to cool to 55 °C and is poured into a suitable mold to form a gel. The gel is then placed in the nitric oxide precursor receiving portion. The reaction solution of buffer 50X TAE is placed at the first and second electrode. An electrical field of 150 VDC is applied to immersed electrodes. The applied electrical field causes the nitric oxide precursor to migrate through the gel and into the reaction chamber to generate nitric oxide. The nitric oxide is dispersed in the headspace above the solution and is swept with the transport gas stream to form the therapeutic gas.

### EXAMPLE 11

Referring to **FIG. 6****,** an electrochemical cell was prepared by fitting a 50 mL glass sample jar with a plastic TEFLON lined lid. Holes are drilled in the lid to accommodate the electrodes. The electrodes are immersed in 1-20% sodium nitrite as a nitric oxide precursor solution and the headspace is swept with the flowing transport gas. A 0.5 to 5 VDC voltage was applied across the cathode and anode. The transport gas transported the generated nitric oxide as therapeutic gas into the optional gas purifier. Depending upon the voltage applied, the nitric oxide precursor concentration and the type of cathode and anode used, nitric oxide levels between 10 and 100 ppm were present in the therapeutic gas. Conditions and results are summarized in Table 4.

**TABLE 4**

| Voltage (volts) | Nitric oxide precursor concentration | Cathode | Anode | ppm of nitric oxide generated in the therapeutic gas |
|---|---|---|---|---|
| 2 | 3% NaNO₂ | Nichrome | Nichrome | 10-100 |
| 2 | 1% NaNO₂ | Nichrome | Copper | |
| 2 | 20% NaNO₂ | Nichrome | Copper | |
| 1-5 | 20% NaNO₂ | Nichrome | Copper | |
| 0.5 | 20% NaNO₂ | | | 26 |
| 5 | 20% NaNO₂ | | | 100 |
| 5 | 1% NaNO₂ | | | 10 |
| 5 | 3% NaNO₂ | | | 100 |

Other embodiments are within the scope of the following claims.

## Claims

1. A method of delivering nitric oxide comprising:
non-electrolytically generating first gas from a nitric oxide precursor that includes a precursor salt including contacting the nitric oxide precursor with a buffer solution to form a mixture, wherein the first gas includes nitric oxide and is substantially devoid of nitrogen dioxide; and
transporting the first gas.

2. The method of claim 1 wherein transporting the first gas includes delivering at least 1 ppm nitric oxide.

3. The method of claim 1 wherein transporting the first gas includes delivering less than 100 ppm of nitric oxide.

4. The method of claim 1 wherein the first gas contains 20 to 60 ppm of nitric oxide.

5. The method of claim 1, wherein the first gas is transported in a transport gas.

6. The method of claim 5 wherein the transport gas includes oxygen, ambient air or a mixture of air and oxygen.

7. The method of claim 1 further comprising passing the first gas through a gas purifier prior to transporting the first gas.

8. The method of claim 7 wherein the gas purifier includes a pyrolizer, a reduction oxidation agent, or a semi-permeable membrane.

9. The method of claim 1 wherein the precursor salt includes a nitrite salt.

10. The method of claim 9 wherein the nitrite salt includes sodium nitrite.

11. The method of claim 1 wherein the nitric oxide precursor includes a nitrite salt selected from a group consisting of potassium nitrite, sodium nitrite, rubidium nitrite, strontium nitrite, barium nitrite, calcium nitrite, copper nitrite and zinc nitrite.

12. The method of claim 1 wherein generating the first gas includes releasing nitric oxide from the precursor for over at least one hour.

13. The method of claim 1 wherein generating the first gas includes releasing nitric oxide from the precursor over a period of time from one hour to seven days.

14. The method of claim 1 wherein generating the first gas includes releasing nitric oxide from the precursor over a period of time from two hours to 24 hours.

## Patentansprüche

1. Verfahren zum Zuführen von Stickoxid, das Folgendes beinhaltet:
nicht elektrolytisches Erzeugen eines ersten Gases von einem Stickoxidvorläufer, der ein Vorläufersalz beinhaltet, das das Inkontaktbringen des Stickoxidvorläufers mit einer Pufferlösung beinhaltet, um ein Gemisch zu bilden, wobei das erste Gas Stickoxid beinhaltet und im Wesentlichen frei von Stickstoffdioxid ist; und
Transportieren des ersten Gases.

2. Verfahren nach Anspruch 1, wobei das Transportieren des ersten Gases das Zuführen von wenigstens 1 ppm Stickoxid beinhaltet.

3. Verfahren nach Anspruch 1, wobei das Transportieren des ersten Gases das Zuführen von weniger als 100 ppm Stickoxid beinhaltet.

4. Verfahren nach Anspruch 1, wobei das erste Gas 20 bis 60 ppm Stickoxid enthält.

5. Verfahren nach Anspruch 1, wobei das erste Gas in einem Transportgas transportiert wird.

6. Verfahren nach Anspruch 5, wobei das Transportgas Sauerstoff, Umgebungsluft oder ein Gemisch aus Luft und Sauerstoff beinhaltet.

7. Verfahren nach Anspruch 1, das ferner das Leiten des ersten Gases durch einen Gasreiniger vor dem Transportieren des ersten Gases beinhaltet.

8. Verfahren nach Anspruch 7, wobei der Gasreiniger einen Pyrolysator, ein Reduktions-Oxidations-Mittel oder eine teildurchlässige Membran beinhaltet.

9. Verfahren nach Anspruch 1, wobei das Vorläufersalz ein Nitritsalz beinhaltet.

10. Verfahren nach Anspruch 9, wobei das Nitritsalz Natriumnitrit beinhaltet.

11. Verfahren nach Anspruch 1, wobei der Stickoxidvorläufer ein Nitritsalz beinhaltet, ausgewählt aus einer Gruppe bestehend aus Kaliumnitrit, Natriumnitrit, Rubidiumnitrit, Strontiumnitrit, Bariumnitrit, Calciumnitrit, Kupfernitrit und Zinknitrit.

12. Verfahren nach Anspruch 1, wobei das Erzeugen des ersten Gases das Freisetzen von Stickoxid von dem Vorläufer für wenigstens eine Stunde beinhaltet.

13. Verfahren nach Anspruch 1, wobei das Erzeugen des ersten Gases das Freisetzen von Stickoxid von dem Vorläufer über eine Zeitperiode von einer Stunde bis zu sieben Tagen beinhaltet.

14. Verfahren nach Anspruch 1, wobei das Erzeugen des ersten Gases das Freisetzen von Stickoxid von dem Vorläufer über eine Zeitperiode von zwei Stunden bis 24 Stunden beinhaltet.

## Revendications

1. Procédé de fourniture d'oxyde nitrique, comprenant :
générer de manière non électrolytique un premier gaz à partir d'un précurseur d'oxyde nitrique qui comprend un sel précurseur, comprenant mettre le précurseur d'oxyde nitrique en contact avec une solution tampon pour former un mélange, dans lequel le premier gaz comprend de l'oxyde nitrique et est sensiblement dépourvu de dioxyde d'azote ; et
transporter le premier gaz.

2. Procédé selon la revendication 1, dans lequel transporter le premier gaz comprend fournir au moins 1 ppm d'oxyde nitrique.

3. Procédé selon la revendication 1, dans lequel transporter le premier gaz comprend fournir moins de 100 ppm d'oxyde nitrique.

4. Procédé selon la revendication 1, dans lequel le premier gaz contient 20 à 60 ppm d'oxyde nitrique.

5. Procédé selon la revendication 1, dans lequel le premier gaz est transporté dans un gaz de transport.

6. Procédé selon la revendication 5, dans lequel le gaz de transport comprend de l'oxygène, de l'air ambiant ou un mélange d'air et d'oxygène.

7. Procédé selon la revendication 1, comprenant en outre passer le premier gaz à travers un purificateur de gaz avant de transporter le premier gaz.

8. Procédé selon la revendication 7, dans lequel le purificateur de gaz comprend un pyrolyseur, un agent d'oxydoréduction, ou une membrane semi-perméable.

9. Procédé selon la revendication 1, dans lequel le sel précurseur est un sel nitrite.

10. Procédé selon la revendication 9, dans lequel le sel nitrite comprend du nitrite de sodium.

11. Procédé selon la revendication 1, dans lequel le précurseur d'oxyde nitrique comprend un sel nitrite sélectionné parmi un groupe consistant en nitrite de potassium, nitrite de sodium, nitrite de rubidium, nitrite de strontium, nitrite de baryum, nitrite de calcium, nitrite de cuivre et nitrite de zinc.

12. Procédé selon la revendication 1, dans lequel générer le premier gaz comprend dégager de l'oxyde nitrique du précurseur pendant une heure au moins.

13. Procédé selon la revendication 1, dans lequel générer le premier gaz comprend dégager de l'oxyde nitrique du précurseur pendant une période de temps d'une heure à sept jours.

14. Procédé selon la revendication 1, dans lequel générer le premier gaz comprend dégager de l'oxyde nitrique du précurseur pendant une période de temps de deux heures à 24 heures.
